# EUROPEAN PATENT SPECIFICATION

(11) **EP 0 339 783 B1**
(45) Date of publication and mention of the grant of the patent: **17.01.1996**
(21) Application number: 89302637.7
(22) Date of filing: 17.03.1989
(51) Int. Cl.: C12Q 1/68

(54) **Detection of yersinia enterocolitica using nucleic acid probes**
Nachweis von Yersinia enterocolitica durch Verwendung von Nucleinsäuresonden
Détection de Yersinia enterocolitica à l'aide de sondes d'acide nucléique

(30) Priority: 18.03.1988 US 169646
(43) Date of publication of application: 02.11.1989
(60) Divisional application: 94118668.6
(73) Proprietor: AMOCO CORPORATION, Chicago Illinois 60680-0703 (US)
(72) Inventor: Shah, Jyotsna S., Nashua, NH 03060 (US); Chan, Samuel W., Newton, MA 02159 (US); Pitman, Theodore B., Lynnfield, MA 01940 (US); Lane, David J., Milford, MA 01757 (US)
(74) Representative: Sheard, Andrew Gregory

(56) References cited:
- EP-A- 0 155 360

## Description

This invention relates to detecting bacteria belonging to the genus Yersinia enterocolitica and more specifically provides nucleic acid probes and compositions along with methods for their use for the specific detection of Yersinia enterocolitica.

The term "Yersinia enterocolitica" as used herein, refers to the bacteria classified as such in Bergey's Manual of Systematic Bacteriology (N.R. Krieg [ed.], 1984, 498-506, Williams & Wilkins). Detection of Yersinia enterocolitica (Y. enterocolitica) is important in various medical and public health contexts. Yersinia enterocolitica infection can cause a variety of symptoms ranging from those resembling a cold to gastroenterocolitis. Under-cooked or uncooked meats are frequently a source of human food-borne infection from these organsims but routine screening is both time consuming and difficult.

It is, therefore, an aspect of the present invention to provide a novel assay system capable of rapidly detecting Yersinia enterocolitica and which is generally applicable to environmental, food or clinical samples.

Pursuant to a standard laboratory method and a method recommended by the F.D.A. (FDA/BAM Bacteriological Analytical Manual, Chapter 11, 6th Edition, 1984, Supplement 9/87', Association of Offical Analytical Chemist), the presence of Yersinia enterocolitica in environmental or dairy specimens (e.g., milk) has been traditionally detected by culturing an appropriately prepared sample on microbiological media under conditions favorable for growth of these organisms. The resulting colonies are then typically examined for morphological and biochemical characteristics, a process that generally is initiated 48 hours after acquisition of the sample and disadvantageously takes between 12-17 days to complete.

It is yet another aspect of the present invention to avoid the disadvantage associated with traditional culturing techniques and to employ nucleic acid probes to detect Yersinia enterocolitica.

It is yet another aspect of the present invention to provide probes which can hybridize to target regions which can be rendered accessible to the probes under normal assay conditions.

While Kohne et al. (1968) Biophysical Journal 8:1104-1118 discuss one method for preparing probes to rRNA sequences they do not provide the teaching necessary to make Yersinia enterocolitica specific probes.

Pace and Campbell (1971) Journal of Bacteriology 107:543-547 discuss the homology of ribosomal ribonucleic acids from diverse bacterial species and a hybridization method for quantitating such homology levels. Similarly, Sogin, Sogin, and Woese (1972) Journal of Molecular Evolution 1:173-184 discuss the theoretical and practical aspects of using primary structural characterization of different ribosomal RNA molecules for evaluating phylogenetic relationships.

Fox, Pechman, and Woese (1977) International Journal of Systematic Bacteriology discuss the comparative cataloging of 16S ribosomal RNAs as an approach to prokaryotic systems. These references, however, fail to relieve the deficiency of Kohne's teaching with respect to Yersinia enterocolitica.

Ribosomes are of profound importance to all organisms because they serve as the only known means of translating genetic information into cellular proteins, the main structural and catalytic elements of life. A clear manifestation of this importance is the observation that all cells have ribosomes.

Ribosomes contain three distinct RNA molecules which, at least in E. coli, are referred to as 5S, 16S, and 23S rRNAs. These names historically are related to the size of the RNA molecules, as determined by sedimentation rate. In actuality, however, they vary substantially in size between organisms. Nonetheless, 5S, 16S, and 23S rRNA are commonly used as generic names for the homologous RNA molecules in any bacteria, and this convention will be continued herein.

Hybridization is traditionally understood as the process by which, under predetermined reaction conditions, two partially or completely complementary single-stranded nucleic acids are allowed to come together in an antiparallel fashion to form a double-stranded nucleic acid with specific and stable hydrogen bonds. The stringency of a particular set of hybridization conditions is defined by the base composition of the probe/target duplex, as well as by the level and geometry of mispairing between the two nucleic acids. Stringency may also be governed by such reaction parameters as the concentration and type of ionic species present in the hybridization solution, the types and concentrations of denaturing agents present, and/or the temperature of hybridization. Generally, as hybridization conditions become more stringent, longer probes are preferred if stable hybrids are to be formed. As a corollary, the stringency of the conditions under which a hybridization is to take place (e.g., based on the type of assay to be performed) will largely dictate the preferred probes to be employed. Such relationships are well understood and can be readily manipulated by those skilled in the art. As a general matter dependent upon probe length, such persons understand stringent conditions to mean approximately 35°C-65°C in a salt solution of approximately 0.9 molar.

As used herein, probe(s) refer to synthetic or biologically produced nucleic acids (DNA or RNA) which, by design or selection, contain specific nucleotide sequences that allow them to hybridize under defined predetermined stringencies, specifically (i.e., preferentially) to target nucleic acid sequences.

A target nucleic acid sequence is one to which a particular probe is capable of preferentially hybridizing.

Still other useful definitions are given as their first use arises in the following text.

In accordance with the various principles and aspects of the present invention, there are provided nucleic acid probes and probe sets comprising DNA or RNA sequences which, under specific hybridization conditions, are capable of detecting the presence of ribosomal RNA (rRNA) molecules of Yersinia enterocolitica but which are not capable, under the same conditions, of detecting the rRNA of other related bacteria which may be present in the test sample.

The invention thus relates to a nucleic acid fragment capable of hybridizing, under predetermined stringency conditions, to an rRNA sequence, or to DNA encoding said rRNA, present in Yersinia enterocolitica and not to rRNA or DNA encoding said rRNA of non-Yersinia enterocolitica, the rRNA Yersinia enterocolitica sequence comprising (using the E.coli numbering convention) the 455 to 477 16S rRNA region.

It relates more particularly to a nucleic acid fragment as aforesaid which is not capable of hybridizing under said conditions to rRNA or DNA encoding said rRNA of Aeromonas sobria, Bacillus cereus, Bacillus subtilis, Brochothrix thermosphacta, Candida albicans, Citrobacter freundii, Corynebacterium xerosis, Corynebacterium diptheriae, Escherichia coli, Escherichia vulneris, Enterobacter agglomerans, Enterobacter cloacae, Klebsiella pneumoniae, Klebsiella oxytoca, Lactobacillus casei, Listeria monocytogenes, Pseudomonas aeruginosa, Rhodococcus egii, Salmonella arizonae, Salmonella cholerae-suis, Salmonella typhi, Serratia odorifera, Shigella boydii, Shigella flexneri, Shigella sonnei, Staphylococcus aureus, Staphylococcus epidermidis, Staphylococcus hominis, Staphylococcus saprophyticus, Streptococcus agalactiae, Streptococcus bovis, Streptococcus faecalis, Streptococcus faecium, Streptococcus lactis, Streptococcus mutans, Streptococcus pneumoniae, or Streptomyces globisporus.

The present invention also features an assay system for the utilization of these probes, the format of which can enhance the aforementioned desirable behavior of the probes. The assay system of the present invention advantageously exhibits the following enhanced performance capabilities with respect to other currently available means for detection of Yersinia enterocolitica:
a) increased sensitivity; i.e., the ability to detect Yersinia enterocolitica in a given sample more frequently than currently available methods;
b) potentially significant reductions in assay cost due to the use of inexpensive reagents and reduced labour;
c) accurate identification of Yersinia enterocolitica even when the biochemically closely related species, Yersinia intermedia is present; and
d) faster results because the test is performed on cultured cells which need not be grown further. Accordingly, the preferred test of this invention advantageously takes only two to four days to provide a result.

It has been discovered that other advantages incurred by directing the probes of the present invention against rRNA include the fact that the rRNAs detected constitute a significant component of cellular mass. Although estimates of cellular ribosome content vary actively growing Yersinia bacteria may contain upwards of 5.0x 10⁴ ribosomes per cell, and therefore 5.0 x 10⁴ copies of each of the rRNAs (present in a 1:1:1 stoichiometry in ribosomes). In contrast, other potential cellular target molecules such as genes or RNA transcripts thereof, are less ideal since they are present in much lower abundance.

A further unexpected advantage is that the rRNAs (and the genes encoding them) appear not to be subject to lateral transfer between contemporary organisms. Thus, the rRNA primary structure provides an organism-specific molecular target, rather than a gene-specific target as would likely be the case, for example of a plasmid-borne gene or product thereof which may be subject to lateral transmission between contemporary organisms.

Additionally, the present invention provides probes to Yersinia enterocolitica rRNA target sequences which are capable of distinguishing two distinct classes of Yersinia enterocolitica. A preferred mixture of two probes can hybridize to the target region in all such Yersinia enterocolitica. Advantageously, these same rRNA target sequences are sufficiently different in most non-Yersinia enterocolitica rRNAs that, under the preferred assay conditions of the present invention, the probe(s) of the present invention hybridize to Yersinia enterocolitica rRNAs and do not generally hybridize to non-Yersinia enterocolitica rRNAs. These probe characteristics are defined as inclusivity and exclusivity, respectively. The discovery that probes could be generated with the extraordinary inclusivity and exclusivity characteristics of those of the present invention with respect to Yersinia enterocolitica was unpredictable and unexpected.

In a particularly preferred embodiment of the invention, an assay method for detecting Yersinia enterocolitica is provided in which bacteria in the sample to be tested are preferably grown for a limited time under conditions which foster rapid and abundant growth of any Yersinia enterocolitica in the sample and which are biased against the growth of many closely related bacteria. Hybridization analysis using the preferred probes of the present invention is then advantageously performed on the sample after this growth period.

The subject-matter for which a protection is sought is featured in appending claims. In this respect, it is to be noted that probe 927 and the complementary DNA sequence to this probe is not part of the present invention.

Further understanding of the principles and aspects of the present invention may be made by reference by way of example to the tables wherein:
- Table 1: Shows alignment of the nucleotide sequences of the preferred probes of the present invention with the nucleotide target sequence including the "core" region from 455 to 477 of Yersinia enterocolitica 16S rRNA (using the E. coli position numbering convention) along with relevant portions of the 16S rRNAs from Escherichia coli (E. coli), Proteus vulgaris (Pr. vulga.), Yersinia enterocolitica type strain ATCC 9610 (Y. ent9610), and Yersinia enterocolitica isolate RF 954 (Y. ent954). RNA sequences are written 5' to 3', probe sequences are DNA and written 3' to 5'. Lower case c in certain of the probes indicates a modified cytosine residue to which a reporter group may or may not be attached depending on the assay form employed. Probes of the 880 series (880, 1062) are shown, along with the "core" region of variation upon which they are based, below their "parent" sequence, Y. ent9610. The 926 series of probes (926, 1111, 1112, 1113, 1063) and the 926 "core" sequence are shown below their "parent" sequence, Y. ent 954. Probe 927 and the "core" variation upon which it is based are shown below its "parent sequence", Y.inte814. Probe 1071 is a detection probe which is designed to be used with any of the 880, 926 or 927 probes.
- Table 2: Exemplifies the inclusivity behavior of the preferred probes toward a representative sampling of Yersinia strains.
- Table 3: Exemplifies the exclusivity behavior of the preferred probes toward a representative sampling of non-Yersinia strains.
- Table 4: Provides inclusivity data related to the preferred liquid hybrid testing format of Example 1.
- Table 5: Provides exclusivity data related to the preferred liquid hybrid testing format of Example 1.
- Table 6: Provides data showing detection of Yersinia enterocolitica in food samples with the preferred probes in a preferred format of the present invention.

There now follows a detailed description of preferred embodiments of the invention.

The first step taken in the development of the probes of the present invention involved identification of regions of 16S and/or 23S rRNA which could potentially serve as target sites for Yersinia enterocolitica specific nucleic acid probes. As a practical matter, it is difficult to predict, a priori, which non-Yersinia enterocolitica organisms might be present in any test sample. Because of the large number of such potential non-Yersinia enterocolitica bacteria, demonstrating exclusivity for any given probe sequence is not only unpredictable but also extremely difficult and laborious. A more rigorous criterion was adopted to obviate the need to know, during initial stages of research and development, what non-Yersinia enterocolitica bacteria might be present in all test samples that ultimately will be screened using the probe. This entailed knowledge of the phylogenetic relationships among Yersinia and between Yersinia and other groups of bacteria. Specifically, an operating but previously unproven hypothesis was adopted that the exclusivity criterion could be satisfied by determining that if a particular target region in Yersinia enterocolitica rRNA, sufficiently different from the homologous region in the rRNA of representative yet close evolutionary relatives of Yersinia enterocolitica, could be identified, then a probe to such a sequence could be used to distinguish between the Yersinia enterocolitica and the relatives by hybridization assay. Based on phylogenetic observations, it was then extrapolated that rRNA sequences of more distantly related organisms, even though their actual identity may not necessarily be known, should be predictably different in a particular region of sequence than the aforementioned close evolutionary relative of Yersinia enterocolitica. However, it cannot be predicted, a priori whether such regions exist or if they do, where within the rRNA such regions will be located.

As our first step in identifying regions of Yersinia enterocolitica rRNA which could potentially serve as useful target sites for nucleic acid hybridization probes, nearly complete nucleotide sequences of the 16S and 23S rRNAs from Yersinia enterocolitica, Yersinia intermedia, Yersinia kristensenii, Yersinia pseudo-tuberculosis, etc. were determined. These were arbitrarily selected as representative of the evolutionary breadth of genus Yersinia. The nucleotide sequences of various portions of the rRNAs were determined by standard laboratory protocols either by cloning (Maniatis et al., 1982, Molecular Cloning: A Laboratory Manual, Cold Spring Harbor Laboratory, New York, 545pp) and sequencing (Maxam & Gilbert, 1977, Proceedings of the National Academy of Science, USA 74:560-564; Sanger et al., 1977, Proceedings of the National Academy of Science, USA 74:5463-5467) the genes which specify the rRNAs, and/or by direct sequencing of the rRNAs themselves using reverse transcriptase (Lane et al., 1985, Proceedings of the National Academy of Science, USA 82:6955-6959).

The identified nucleotide sequences were compared to one another and to other available rRNA nucleotide sequences, in particular to those of closely related bacteria such as Morganella, Proteus, Salmonella, Escherichia, and Pasteurella. The preferred region of sequence shown in Table 1 was identified as potentially exhibiting useful exclusivity characteristics with respect to these species.

Further experimental testing of each nucleic acid probe was conducted to rigorously demonstrate whether the desired characteristics discussed above could indeed be obtained, namely: 1) adequate exclusivity to all even closely related non-Yersinia enterocolitica organisms, 2) useful inclusivity patterns with respect to Yersinia enterocolitica strains, and 3) accessibility of the target regions under various assay conditions that might actually be employed. Because of the extremely large number of organisms potentially relevant to defining exclusivity (presently ca. 22 enteric genera, comprised of some 69 species and 29 unspeciated 'biogroups', Farmer et al., 1985) and inclusivity (on the order of 8 species and biogroups of Yersinia) characteristics of test probes, an iterative strategy was adopted to test and refine potential probes. The probes were conveniently synthesized by standard phosphoramidite (Caruthers, M.H. et al. [1983], in Gene Amplification and Analysis, eds. Papas, T.S., Rosenberg, M., Charikjian, J.G., Pub. Elsevier, New York, Vol. 3 pp.1-26) techniques on an Applied Biosystems instrument.

"Dot blot" analysis, in accordance with well known procedures, was employed to preliminarily test the inclusivity and exclusivity properties of these first generation probes. As is known, dot blot analysis generally involves immobilizing a nucleic acid or a population of nucleic acids on a filter such as nitrocellulose, nylon, or other derivatized membrane which can be readily obtained commercially, specifically for this purpose. Either DNA or RNA can be easily immobilized on such a filter and subsequently can be probed or tested for hybridization under any of a variety of nucleic acid hybridization conditions (i.e. stringencies) with nucleotide sequences or probes of interest. Under stringent conditions, probes whose nucleotide sequences have greater complementary to the target sequence will exhibit a higher level of hybridization than probes containing less complementarity. For the oligonucleotide probes described herein, (i.e., 30-36 nucleotides in length and of average base composition) hybridization to rRNA targets at 60°C, for 14-16 hours (in a hybridization solution containing 0.9 M NaCl, 0.12 M Tris-HCl, pH 7.8, 6 mM EDTA, 0.1 M KP04, 0.1% SDS, 0.1% pyrophosphate, 0.002% ficoll, 0.02% BSA, and 0.002% Polyvinylpyrrolidine) followed by three, 15 minutes post-hybridization washes at 60°C to remove unbound probes (in a solution containing 0.03 M NaCl, 0.004 M Tris-HCl, pH 7.8, 0.2 mM EDTA and 0.1% SDS), would be sufficiently stringent to produce the levels of specificity and sensitivity demonstrated in the tables and examples. Techniques are also available in which DNA or RNA present in crude (unpurified) cell lysates can be immobilized without having to first purify the nucleic acid in question (referred to herein as cytodots, see for example Maniatis, T., Fritsch, E.F. and Sambrook, J. (1982) Molecular Cloning, a Laboratory Manual). This latter approach significantly decreases the amount of effort required to screen for particular nucleotide sequences which may be present in the nucleic acids of any particular organism and, moreover, is advantageously amenable to the mass screening of large numbers of organisms. It, therefore, is the method of choice for exclusivity and inclusivity screening of potential nucleic acid hybridization probes vs large numbers of organisms.

A list of non-Yersinia bacteria which exemplify the type of bacteria that may be present in potentially Yersinia enterocolitica containing samples is given in Table 3. Note that these also represent many of the genera most closely related to Yersinia. As discussed above, a probe which demonstrates good exclusivity characteristics to such a broad representation of bacteria can reasonably be predicted to behave similarly to a much broader list of more distantly related enteric organisms.

Several other considerations also affect optimal design characteristics of a probe sequence. The first is consideration of the geometry of the probe with respect to itself (i.e., intramolecular interactions). It has been discovered that potentially useful target region of 16S and 23S rRNAs most often are located in regions that exhibit a substantial possibility for self complementarity. As a result, probes to these regions can also exhibit self-complementarity. Because potential interactions between the probe and target sequences are governed by the same types of parameters that govern the intramolecular annealing of the target or probe sequences to themselves, it is possible, particularly under solution hybridization conditions, that self-complementary probes can render themselves inaccessible for hybridization to their target sequences. Thus, one important aspect of the probe design is to minimize such self-complementarity. This necessitates making a compromise between maximum utilization of Yersinia enterocolitica-specific sequences and acceptable probe geometry.

A second consideration in probe design arises with respect to the inclusivity criterion. The preferred probe will be one which, while displaying appropriate exclusivity behavior, can also hybridize to the rRNA(s) of all desired Yersinia enterocolitica bacteria. Because the species Yersinia enterocolitica itself is comprised of bacteria which exhibit significant phenotypic and genotypic (including, as disclosed below, rRNA) diversity, the design of such an "ideal" probe is greatly complicated. In practice, rather than searching for a single "universal" Yersinia enterocolitica probe, a set of Yersinia enterocolitica-specific probes is more preferably sought, each of which exhibits appropriate exclusivity along with a useful level of inclusivity. In aggregate, a preferred set of probes should ideally detect most or all Yersinia enterocolitica and no non-Yersinia enterocolitica bacteria. In such a set, for example, one probe may detect all but one or a few important Yersinia enterocolitica strains, and another probe may hybridize only to those few Yersinia enterocolitica strains missed by the first probe. Thus, although the probes disclosed below are characterized on an individual basis with respect to inclusivity characteristics, it should be recognized that the concept of "sets" of specific probes as detailed above is preferably considered in determining the importance of individual probes and in constructing assay kits.

The final steps of probe design and analysis ideally comprise testing real (e.g., food/clinical/environmental) samples and then selecting suitable probes for a final probe set so that the desirable properties are optimized under real assay conditions.

### Probes

The foregoing probe selection strategy yielded a number of probes useful for identifying Yersinia enterocolitica bacteria in samples. As outlined in the Brief Description, Table 1 gives the probe sequences, aligned upon their target sites in the rRNAs of representative Yersinia enterocolitica strains. The "core" regions of nucleotide sequence differences within the target sites of Yersinia enterocolitica rRNA and representative non-Yersinia enterocolitica rRNA, which are the basis of the desirable discriminatory behavior of the probes, are also shown.

Three series of probes were developed: the "880" series and the "926" series, named after the first probe developed in each series and Yersinia enterocolitica-specific, and the "927" probe which is Yersinia intermedia specific. The members of each series differ somewhat in length, base composition, and "geometry" with respect to positioning of Yersinia enterocolitica-specific nucleotides in the 16S rRNA target sequence. Under given defined hybridization conditions, one probe from each series (880 and 926) will perform optimally with respect to inclusivity and exclusivity behavior. Probes from both series are capable of hybridizing, with exceptional exclusivity, to Yersinia enterocolitica under appropriately selected hybridization conditions. While either probe of the 880 or 926 series of the present invention demonstrates acceptable inclusivity for a specific subset of Yersinia enterocolitica, the ideal probe composition comprises a mixture of two probes, one from each series, in order to detect all Yersinia enterocolitica in all known cases. The probes are ideally selected in accordance with the particular assay format selected and its associated stringency characteristics.

Table 2 shows the hybridization behavior of the probes toward rRNA target from representative species of Yersinia. The 880 series of probes was based on the 16S rRNA sequences of the Yersinia enterocolitica type strain ATCC 9610 and, as can be seen in Table 2, hybridizes strongly to that strain in addition to a number of other Yersinia enterocolitica strains. The 926 series of probes was based on the 16S rRNA sequence of Yersinia enterocolitica strain RF 954 (isolated in house but equivalent to human isolate E641 [D.A. Schiemann, Montana State University, Bozeman Montana 59717]). As shown in Table 2, the 926 probes hybridize strongly to a subset of Yersinia enterocolitica quite distinct from those to which the 880 probes hybridize. No other species of Yersinia are detected by either of the probes except for two strains of Yersinia kristensenii, which are detected by the 926 probes. This minor deviation is unimportant from a clinical perspective. Note also that both probes distinguish between Yersinia enterocolitica and the biochemically difficult to distinguish, Yersinia intermedia.

Yersinia intermedia can be specifically distinguished from Yersinia enterocolitica by the 927 probe shown in Table 1.

Table 3 shows the hybridization behavior of the probes versus "cyto blots" of various non-Yersinia bacteria. In this experiment the probes were radioactively labelled with Phosphorous-32 for detection and quantitation. Little or no cross-hybridization of the shorter probe versions of each probe series was observed under the hybridization conditions employed in this experiment. These conditions comprised hybridizing at 60°C for 14 - 16 hours in the hybridization solution previously described. Only the longest, less preferred probe version of the 926 probe series began to show limited reaction with non-Yersinia shown and thus defines an upper limit of optional probe length for use with these hybridization stringents. The more preferable intermediate length probe versions demonstrated the desired pattern of reactivity at the given hybridization condition. It will be readily recognized, however,that as assay formats of higher stringency are employed, the use of longer versions of the probes become more desirable since their level of cross-reactivity will decline while their level of sensitivity (hybridization efficiency) remains high.

### Example 1 - General: A Homopolymer Capture, Dual Probe, Liquid Hybridization Format

Cultures containing Yersinia and/or non-Yersinia bacteria are grown in appropriate broth, then the nucleic acids are released by any of a number of appropriate lysis agents (e.g., NaOH, Guanidine salts, detergent, enzymatic treatment, or some combination of the aforementioned). Hybridization is carried out with two different probes or probe sets at least one of which, but not necessarily both, must be specific for the organism to be detected. In this example, the Yersinia enterocolitica specific "capture" probes, 880 and 926, are enzymatically tailed with 20-200 deoxyadenosine (dA) residues at their 3' termini, and the reporter probe, 1071, is labeled either chemically or enzymatically with radioactive Phosphorous (P-32) or other small ligand (e.g., fluorescein or biotin) which is used to detect the capture target molecules.

Generally, following cultivation/enrichment, bacteria present in the test samples are transferred in small aliquots to test tubes. The bacteria are lysed, the capture and detection probes are added, and hybridization is allowed to proceed in an appropriate solution at an appropriate temperature such as those already described. The solution containing the target/probe complex then is brought into contact with a surface containing bound deoxythymidine (dT) homopolymer 15-3000 nucleotides in length, under conditions that will allow hybridization between the dA and dT. In this example, the dT is bound to a plastic "dipstick" which is submerged in the target/probe solution. If Yersinia enterocolitica ribosomal RNA was present in the test sample, the dA tailed, Yersinia enterocolitica- specific capture probes would have hybridized to the target rRNAsequences present and, in turn, would be captured onto the dipstick. Unhybridized nucleic acids and cellular debris are washed away, leaving the captured DNA-RNA complex attached to the surface via the dA-dT duplex. The reporter probe also is bound to the dipstick via the chain of interactions - Capture surface-dT: dA-Capture probe:Target:Reporter Probe - only if the correct target nucelic acid is present. The bound, ligand derivatized (e.g., fluoresceinated) reporter probe then is detected by the addition of a ligand binding-enzyme complex (e.g., anti-fluroscein:alkaline phosphatase, streptavidin:horse radish peroxidase, etc.). Following incubation under conditions permitting specific binding of the detection complex, washing to remove non-bound enzyme, addition of chromogenic substrate and subsequent color development (typically 20-30 minutes), and the optional addition of color-termination solution, the developed color is measured colorimetrically. This reading (typically in the range of 0.2 - > 2.0 0.D. units) is compared to the negative control levels, a threshold or cutoff value is established, and a determination of the "significance" of the experimental levels is made. Tables 4 and 5 show the results of one such experiment, using pure culture of various Yersinia (Table 4) and non-Yersinia (Table 5) bacteria.

### Example 1 - Specific

For dairy or meat product samples, 25 g of the food sample was added to 225 ml of Yersinia enrichment broth (PSBB, peptone-sorbitolbile-broth: containing Sodium phosphate [dibasic] 8.23 g, Sodium phosphate [mono-basic] 1.2 g, Bile salts #3 1.5 g, Sorbitol 5 g, Peptone 10 g, Distilled water 1 liter) in order to obtain primary enrichment for Yersinia enterocolitica in the sample. The mixture was homogenized using a blender or stomacher as appropriate for the particular sample type, and then incubated in a bottle for 20-28 hours, most preferably 24 hours, at between 20°C and 35°C, most preferably at 35°C.

For environmental testing, a swab or other environmental sample was placed in a flask or bottle containing 25 ml (or larger volume if required) PSBB, and incubated for 20-28 hours, most preferably 24 hours, at between 20-35°C, most preferably at 35°C.

Secondary enrichment for all sample types was then performed. The primary enrichment culture was removed from incubation and mixed well. 1 ml (or 10 ml) of the primary enrichment culture was transferred to a test tube or bottle containing 10 ml (or 100 ml) PSBB and incubated for 24 + or - 4 hours at 20-35°C, most preferably at 35°C.

0.45 ml of the cultured cells were treated with 0.05 ml lysis solution (NaOH, 1.25 N) by incubation at room temperature (15-30°C) for 5 minutes The bacterial lysates were neutralized with the addition of 0.1 ml of neutralization buffer (KPO₄:NaPO₄(1:1), 4 M). The nucleic acids released from each sample was detected by addition of 0.05 ml specific capture and detector probe sets (containing between 1.4-2.8 microgram/ml of preferred capture probe set 880/926 although other combinations from the 880/926 series of probes may be used; and, 2-4 microgram/ml of detector probe 1071). A set of capture dipsticks were placed into the test tubes containing the bacterial lysates and the specific probes sets. The contents were incubated in a 65°C water bath for 60 minutes to enable hybridization of specific capture and reporter probes to target nucleic acids and the capture of these specific DNA/rRNA hybrids to the dipsticks.

After hybridization, the dipsticks were washed by dipping the dipsticks in a wash basin containing enough wash solution to cover the active part of the dipstick (50 mM Tris, pH 7.5, 150 mM NaCl, 2 mM EDTA, and 0.1% Tween® 20) for 1 minute. This process was then repeated in fresh solution.

The washed dipsticks were removed from the wash basin, blotted dry with absorbent paper, placed into a set of test tubes containing 0.75 ml antibody-enzyme conjugates (anti-fluorescein-horse radish peroxidase diluted in wash buffer), and allowed to incubate at room temperature for 20 minutes.

After allowing the antigen-antibody reaction to occur, the dipsticks were removed from the test tubes, washed and blotted in the same manner as described in the preceding two paragraphs. The dipsticks were placed into a set of labelled test tubes containing substrate-chromogen mixtures and allowed to incubate at room temperature for 20-30 minutes. The dipsticks were then removed and the colour development step terminated by the addition of 0.25 ml 4N sulphuric acids. The optical density of the samples was measured colorimetrically.

Generally, the average of three negative controls (prepared from a selected non-Yersinia enterocolitica bacterium) was obtained and a value equal to 2.5X the average O.D. of the three negative controls chosen as the cutoff value. Sample tubes with higher O.D. value were considered positive for Yersinia enterocolitica, those with lower O.D. values indicated the absence of Yersinia enterocolitica. Results are shown in Table 6.

While the description of the invention has been made with reference to detecting rRNA, it will be readily understood that the probes described herein and probes complementary to those described herein will also be useful to detect the genes (DNA) encoding the rRNA and accordingly, such probes are to be deemed equivalents to the described probes and encompassed within the spirit and scope of the present invention and the appended claims.

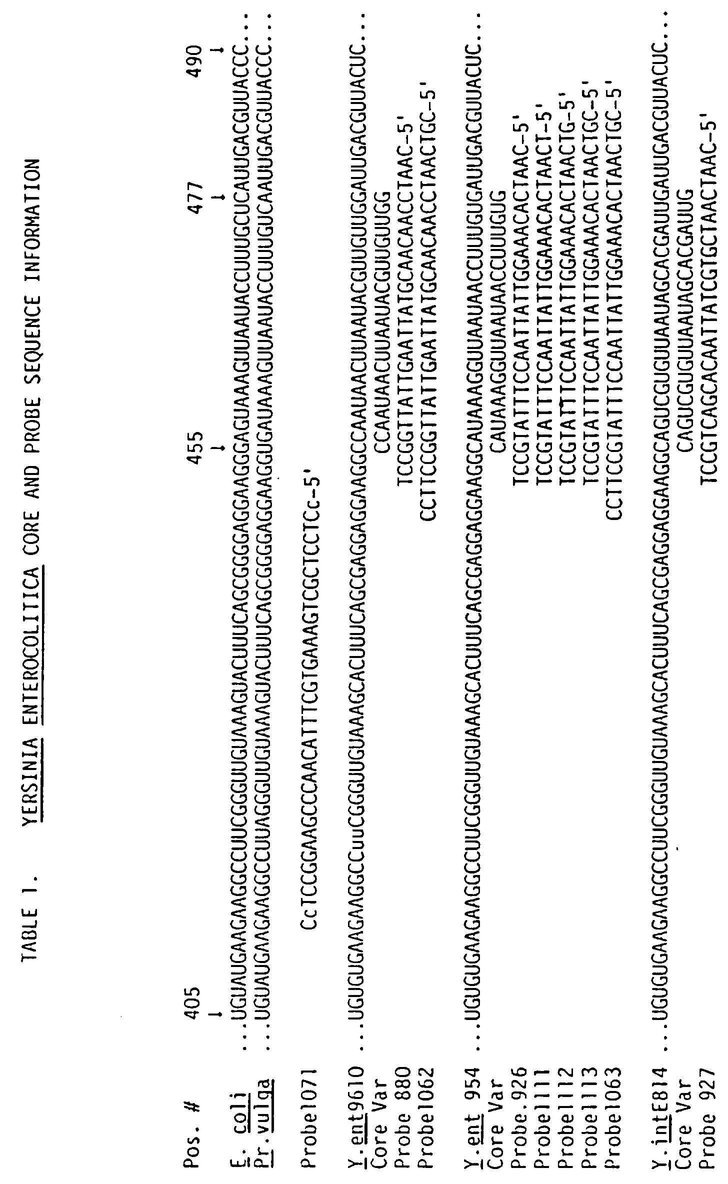

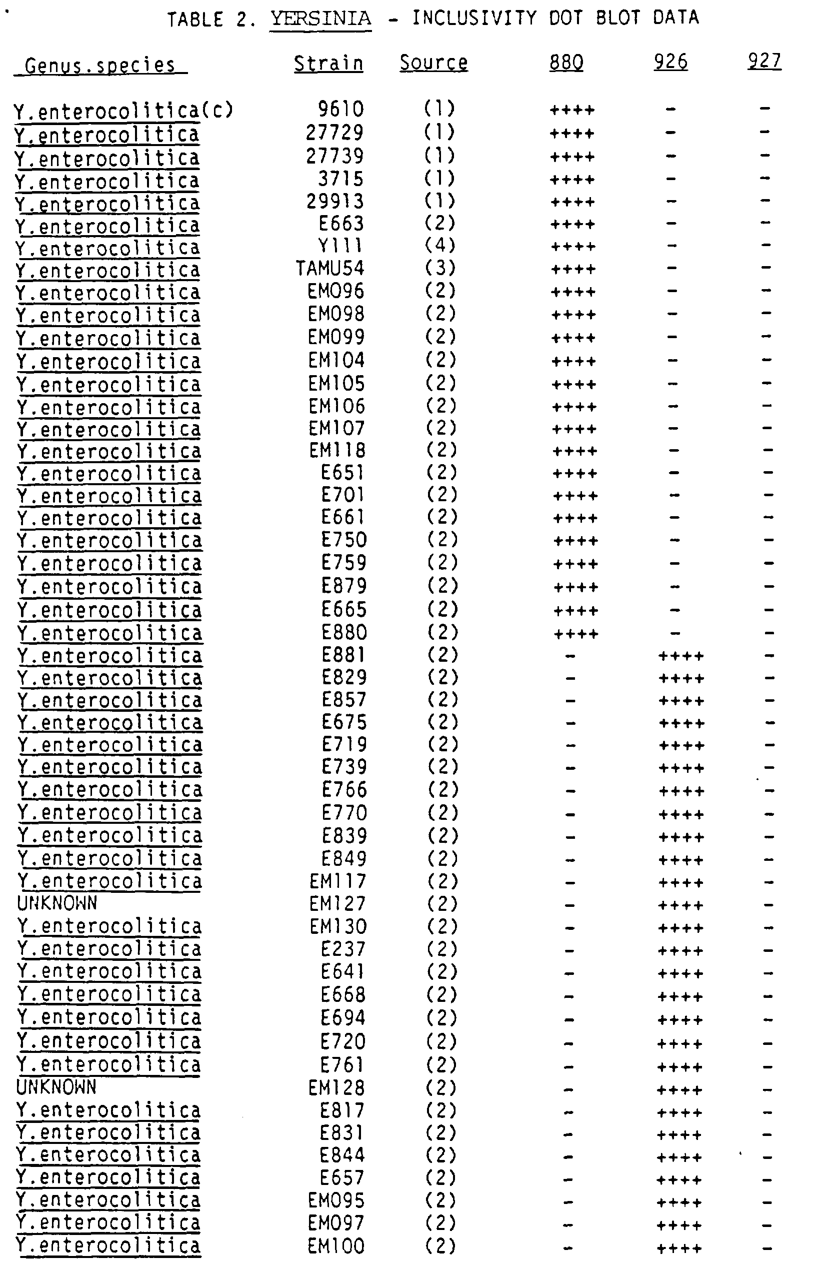

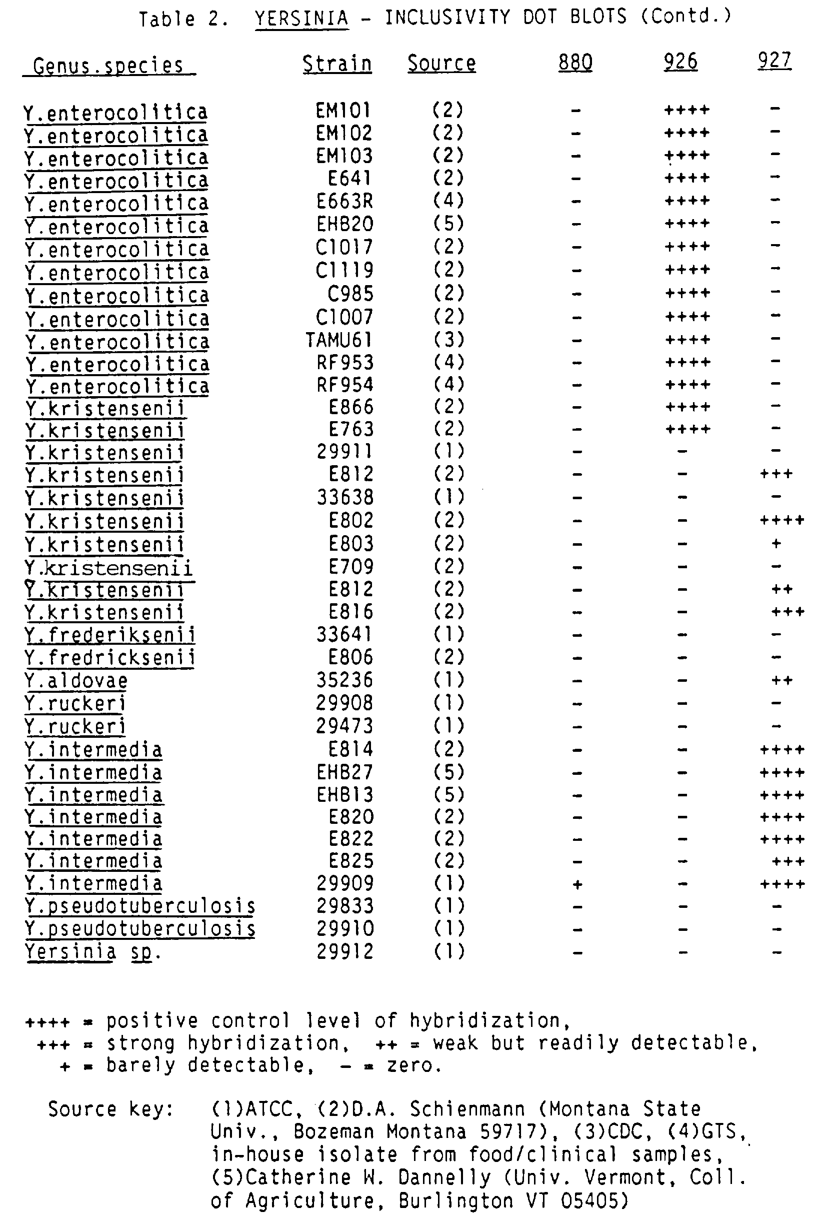

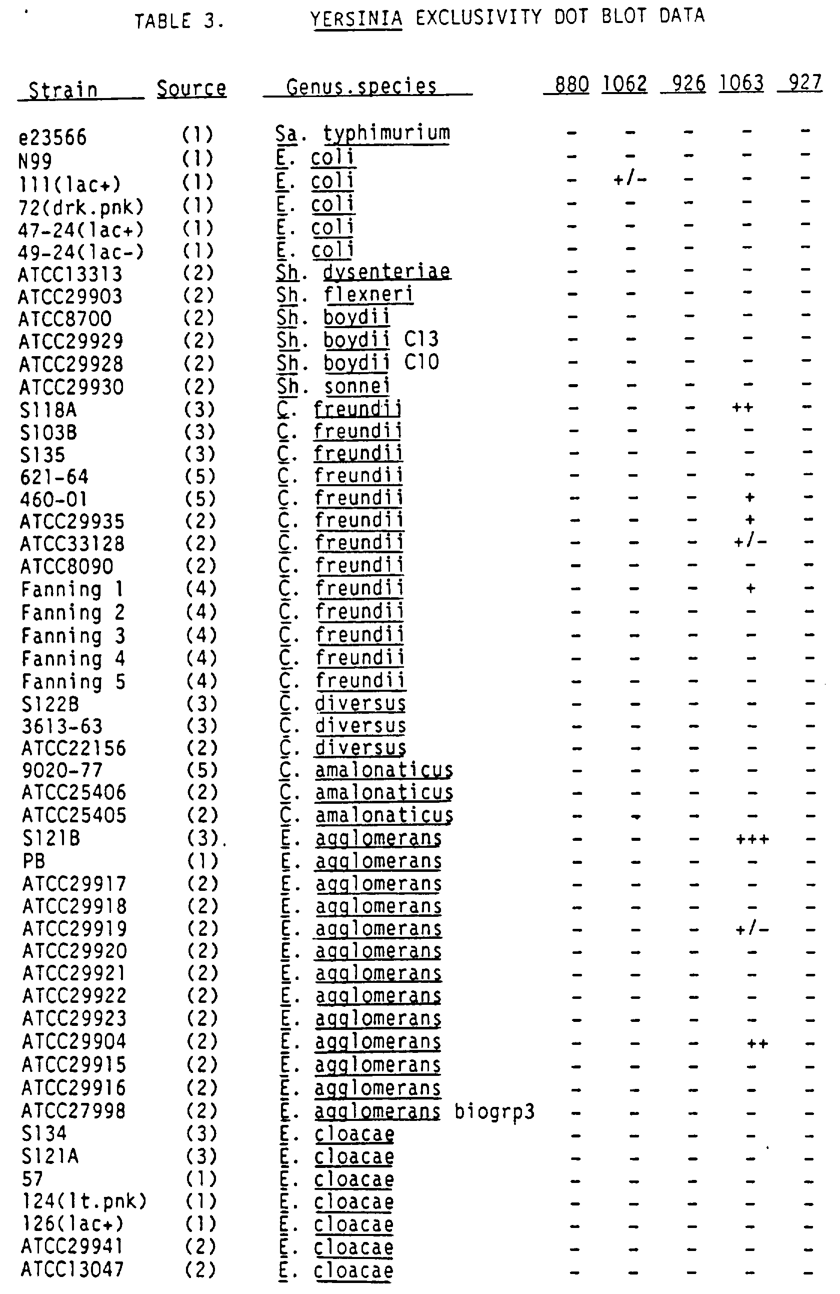

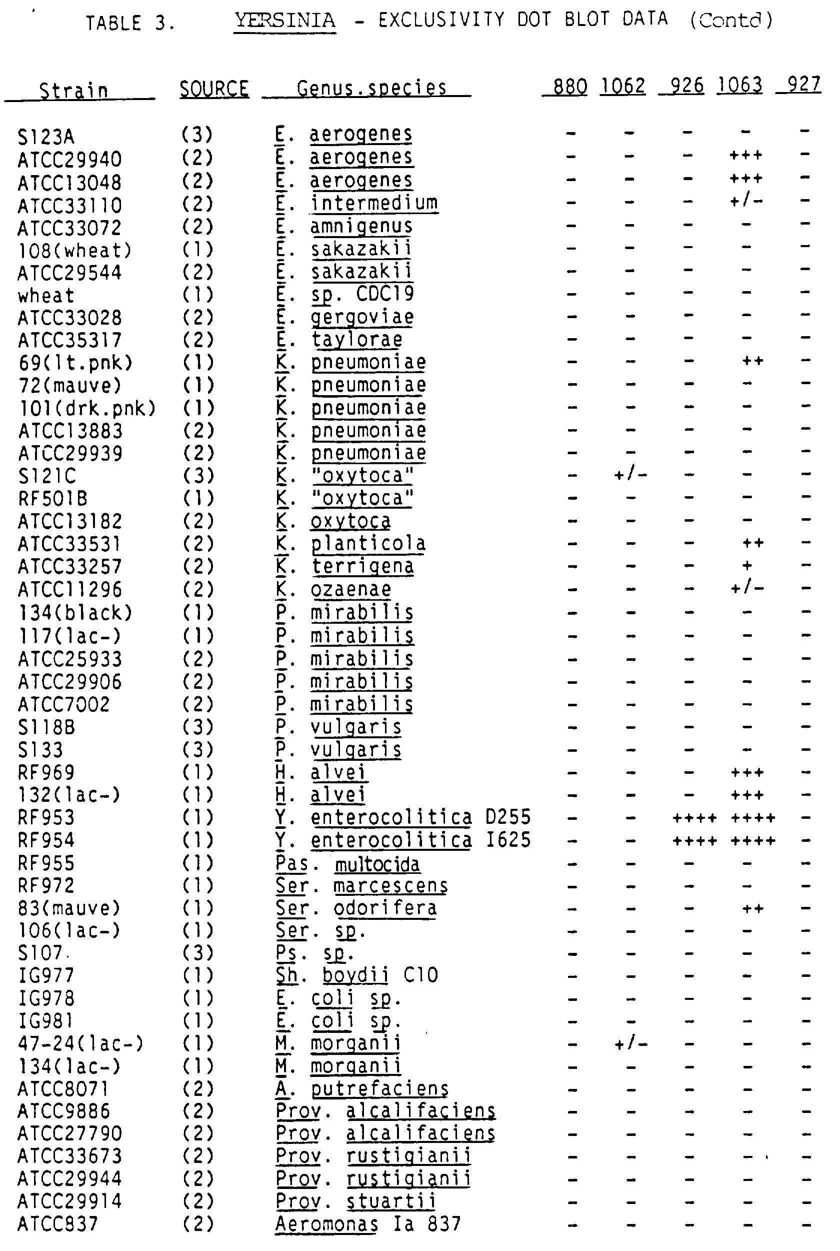

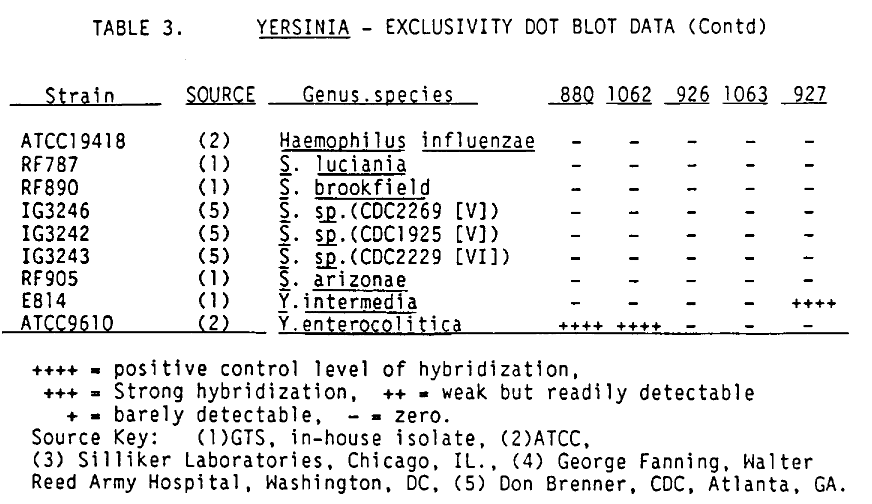

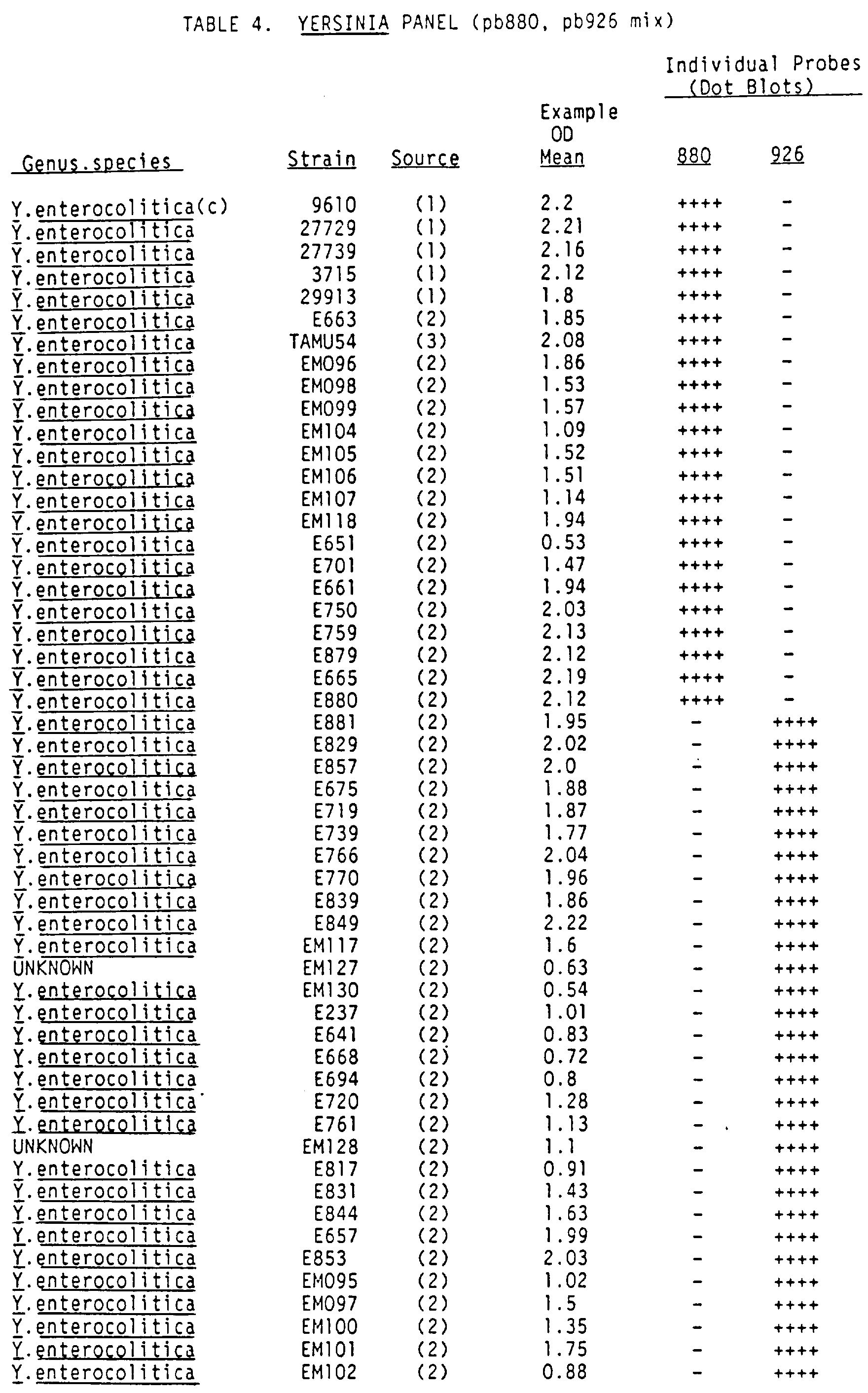

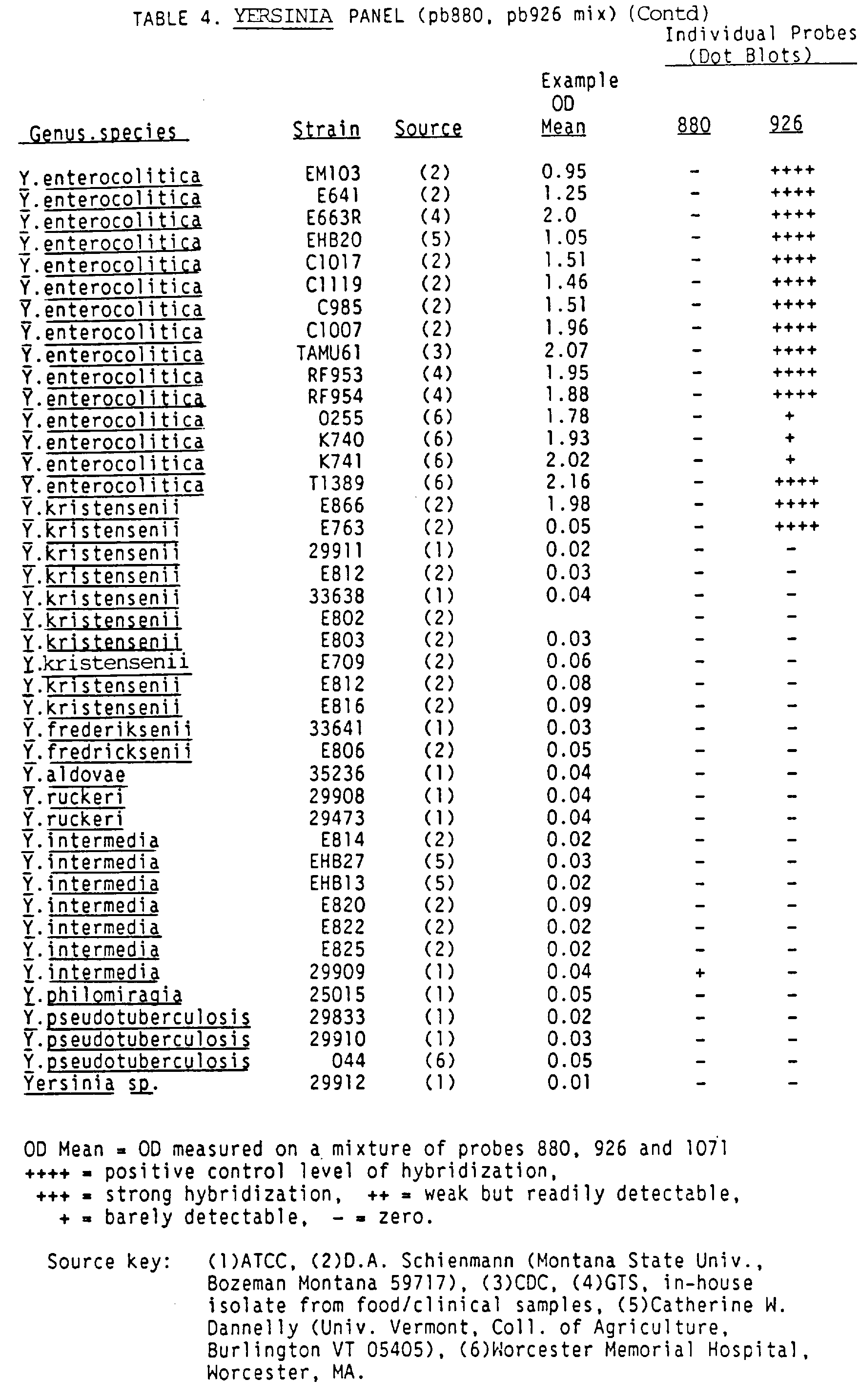

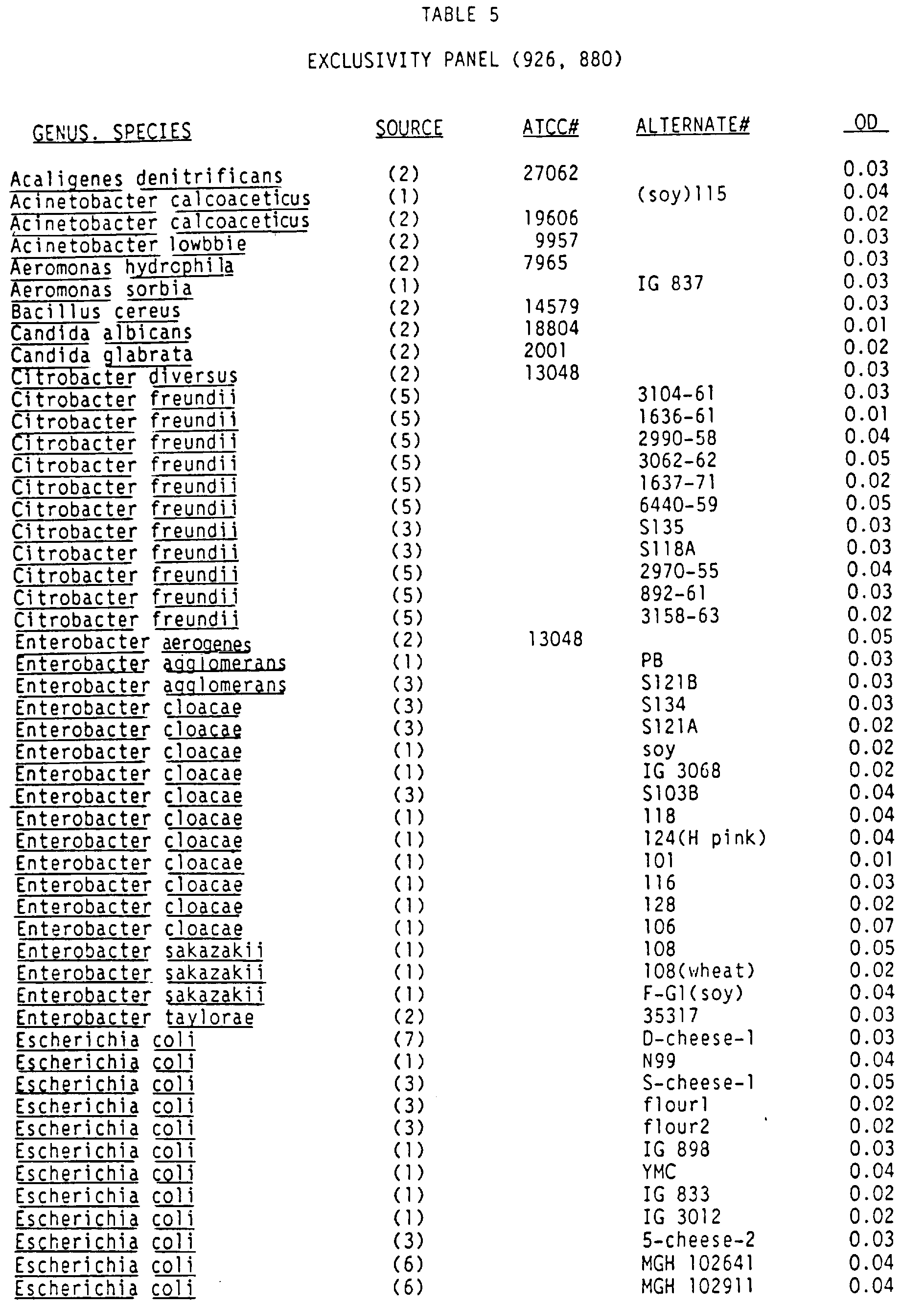

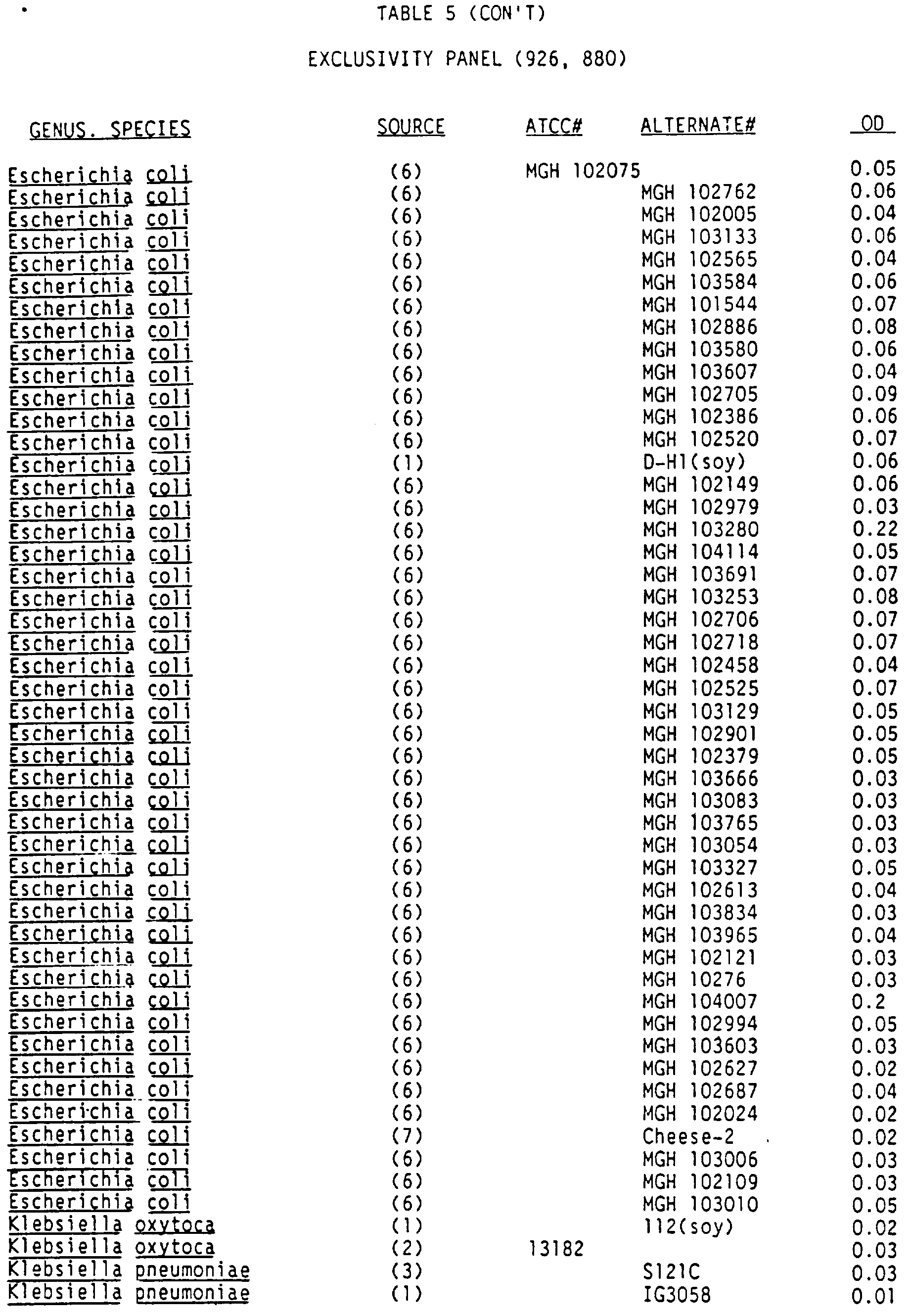

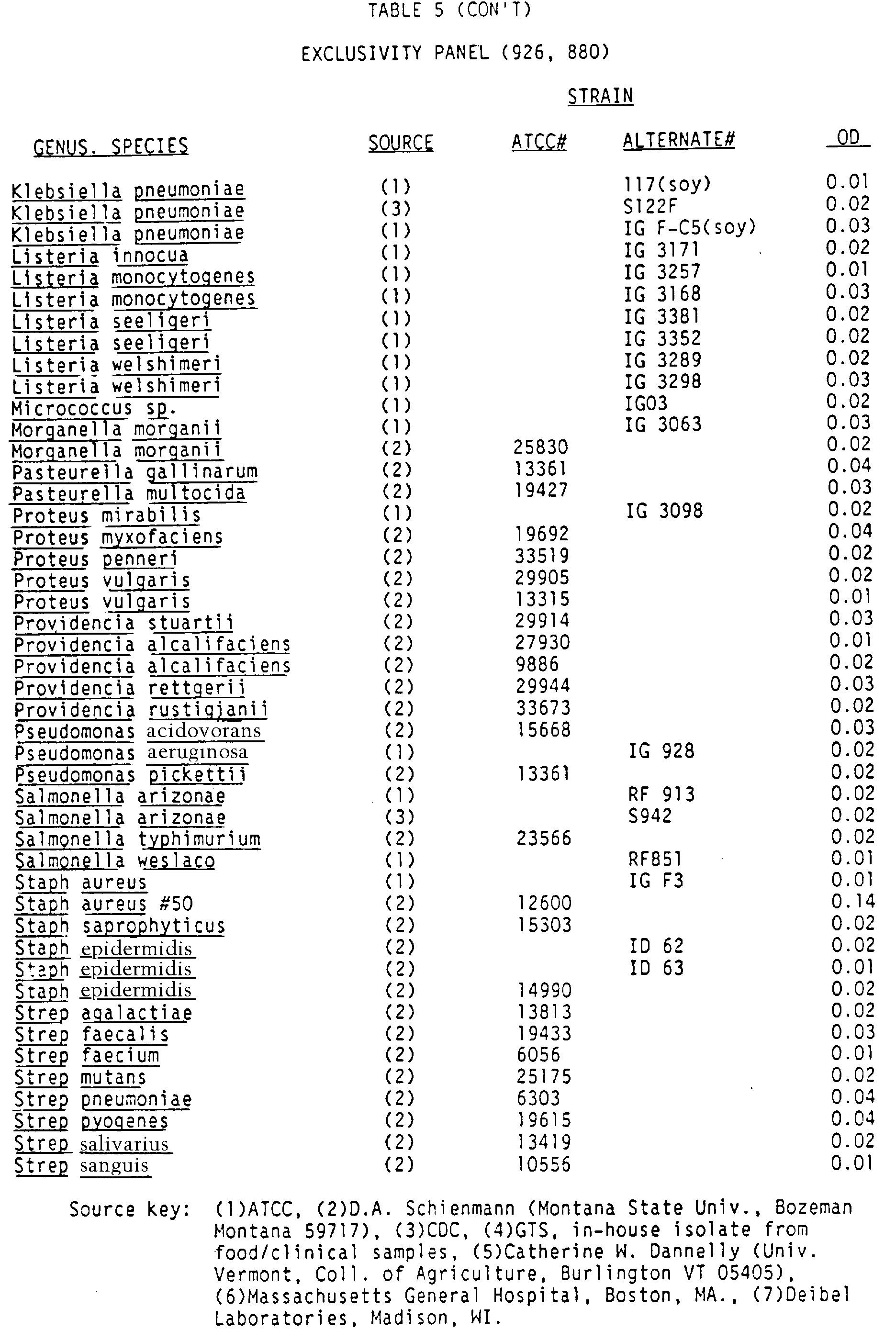

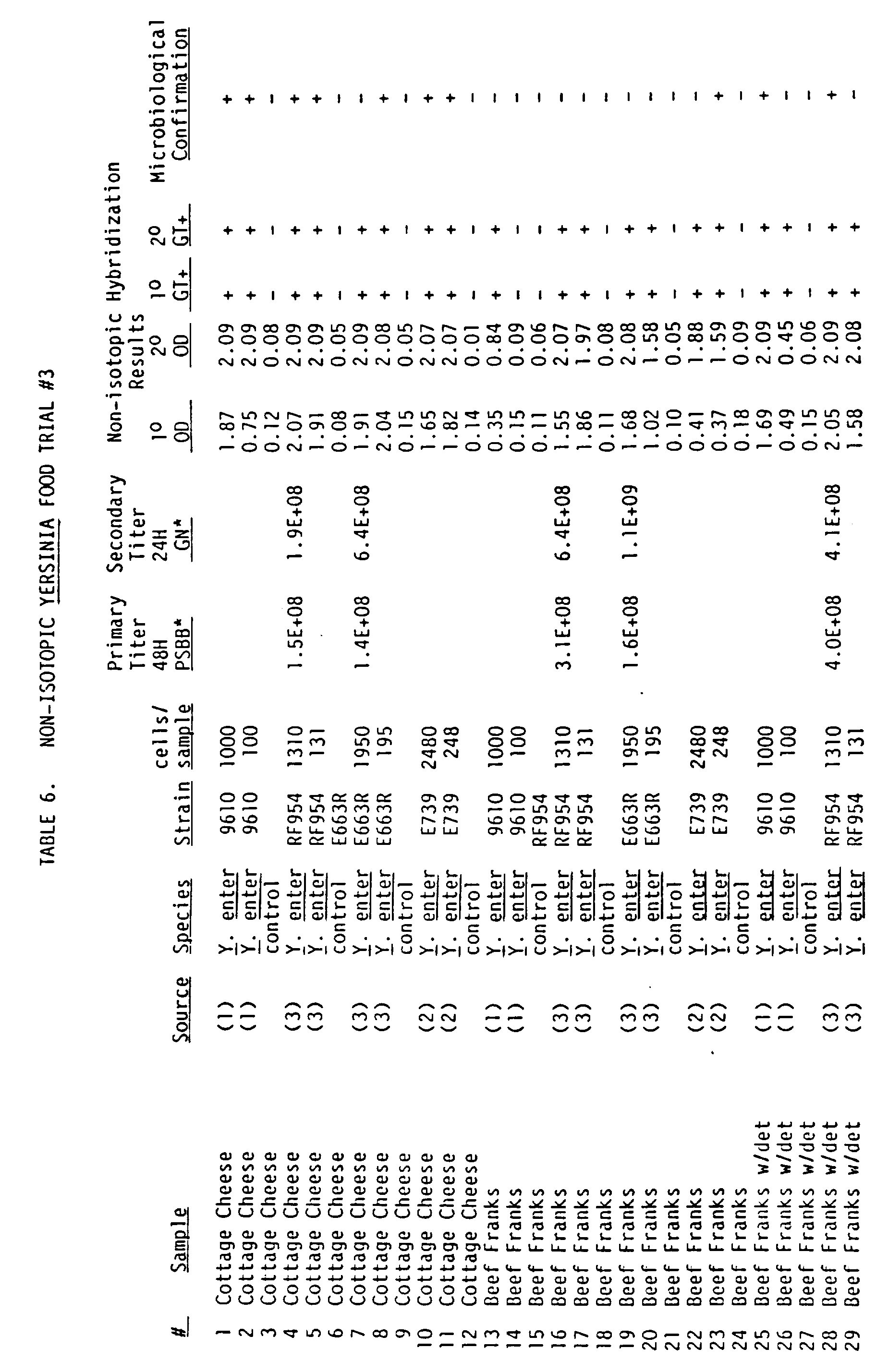

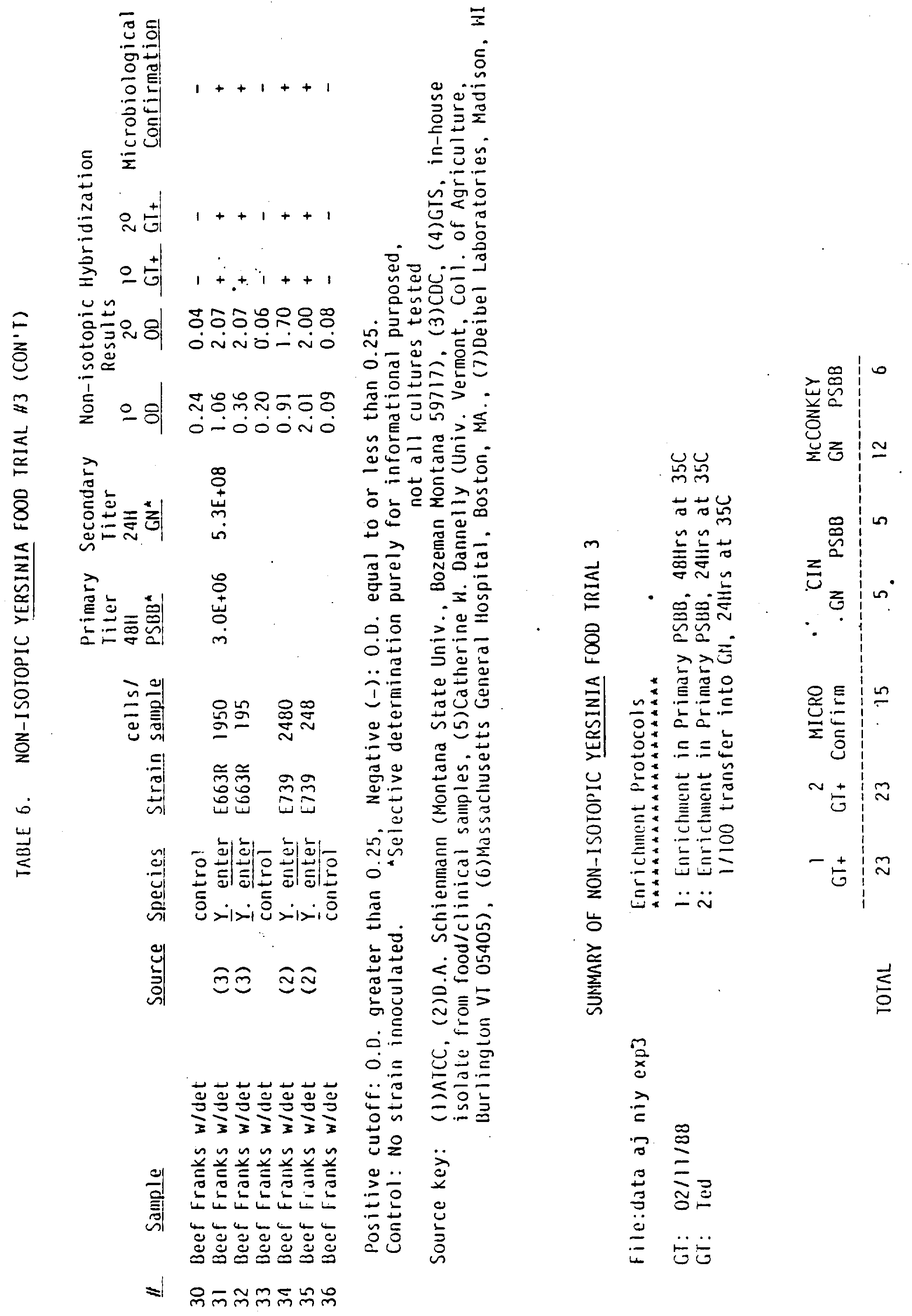

## Claims

1. A nucleic acid fragment capable of hybridizing, under predetermined stringency conditions, to an rRNA sequence, or to DNA encoding said rRNA, present in Yersinia enterocolitica and not to rRNA or DNA encoding said rRNA of non-Yersinia enterocolitica, the rRNA Yersinia enterocolitica sequence comprising (using the E.coli numbering convention) the 455 to 477 16S rRNA region.

2. A nucleic acid fragment as claimed in Claim 1 which is not capable of hybridizing, under said conditions, to rRNA or DNA encoding said rRNA of Aeromonas sobria, Bacillus cereus, Bacillus subtilis, Brochothrix thermosphacta, Candida albicans, Citrobacter freundii, Corynebacterium xerosis, Corynebacterium diptheriae, Escherichia coli, Escherichia vulneris, Enterobacter agglomerans, Enterobacter cloacae, Klebsiella pneumoniae, Klebsiella oxytoca, Lactobacillus casei, Listeria monocytogenes, Pseudomonas aeruginosa, Rhodococcus egii, Salmonella arizonae, Salmonella cholerae-suis, Salmonella typhi, Serratia odorifera, Shigella boydii, Shigella flexneri, Shigella sonnei, Staphylococcus aureus, Staphylococcus epidermidis, Staphylococcus hominis, Staphylococcus saprophyticus, Streptococcus agalactiae, Streptococcus bovis, Streptococcus faecalis, Streptococcus faecium, Streptococcus lactis, Streptococcus mutans, Streptococcus pneumoniae, or Streptomyces globisporus.

3. A nucleic acid fragment as claimed in Claim 1 or 2, which is capable of hybridizing under hybridizing conditions to probe 880, 1062, or an oligomer comprising 880 and having a length longer than 880 but less than 1062, or to a sequence complementary to any of the foregoing.

4. A nucleic acid fragment as claimed in Claim 1 or 2, which is capable of hybridizing under hybridizing conditions to probe 926, 1111, 1112, 1113, 1063 or to a sequence complementary to any of the foregoing.

5. A nucleic acid fragment according to any preceding claim wherein the 16S rRNA Yersinia enterocolitica sequence comprises either:
(a) the 449 to 484 region of Y. enterocolitica 9610 which is: 5'-GGAAGGCCAAUAACUUAAUACGUUGUUGGAUUGACG-3'; or
(b) the 449 to 484 region of Y. enterocolitica 954 which is:

5'-GGAAGGCAUAAAGGUUAAUAACCUUUGUGAUUGACG-3'

6. A set of nucleic acid fragments comprising:
a) a first nucleic acid fragment as defined in Claim 1 which is capable of hybridizing to rRNA of a first set of Yersinia enterocolitica and not to rRNA of a second set of Yersinia enterocolitica or to rRNA of non-Yersinia enterocolitica; and
b) a second nucleic acid fragment, also as defined in Claim 1 but different from that in (a) that is capable of hybridizing to rRNA of said second set of Yersinia enterocolitica and not to rRNA of non-Yersinia enterocolitica.

7. A set of nucleic acid fragments as claimed in Claim 6 wherein the fragments are together capable of hybridizing to at least 95% of Yersinia enterocolitica species.

8. A set of nucleic acid fragments according to claim 6 or 7 wherein:
(a) the first fragment is capable of hybridizing the 449 to 484 16S rRNA region of Y. enterocolitica 9610 (sequence A) but not to the 449 to 484 16S rRNA region of Y. enterocolitica 954 (sequence B) ; and
(b) the second fragment is capable of hybridizing to sequence B but not to sequence A.

9. A set of nucleic acid fragments according to claim 8 wherein the first fragment comprises probe 1062 and the second fragment comprises 1063.

10. A method for detecting the presence of Yersinia enterocolitica in a sample, the method comprising:
(a) contacting a nucleic acid fragment as defined in any of Claims 1 to 5 or a set of nucleic acid fragments as claimed in any of Claims 6 to 9 with the said sample under conditions that allow at least one of said fragments to hybridize to rRNA of Yersinia enterocolitica, if present in said sample, to form hybrid nucleic acid complexes, and
(b) detecting the hybrid complexes as an indication of the presence of Yersinia enterocolitica in the sample.

11. A method as claimed in Claim 10 wherein the nucleic acid fragment is capable of hybridizing, under hybridizing conditions, to probe 926, 1111, 1112, 1113, 1063, or to a sequence complementary to any of the foregoing.

12. A method as claimed in Claim 9 wherein the fragments comprise a mixture of at least one fragment capable of hybridizing, under stringent conditions, to probe 926, 1111, 1112, 1113, 1063, or a sequence complementary to any of the foregoing; and at least one other fragment capable of hybridizing under stringent conditions to probe 926, 1111, 1112, 1113, 1063, or a sequence complementary to any of these.

13. An assay kit for detecting Yersinia enterocolitica comprising a nucleic acid fragment as claimed in any of Claims 1 to 5 or a set of nucleic acid fragments as claimed in any of Claims 6 to 9 packaged in at least one container, and instructions for utilizing the said nucleic acid fragment(s) for detecting Yersinia enterocolitica.

## Patentansprüche

1. Nucleinsäurefragment, das unter vorbestimmten Stringenzbedingungen mit einer rRNA-sequenz oder mit die rRNA codierender DNA, die in Yersinia enterocolitica vorliegt, hybridisieren kann, und nicht mit rRNA oder die rRNA codierender DNA von nicht-Yersinia enterocolitica, wobei die rRNA-Sequenz von Yersinia enterocolitica (unter Verwendung der E. coli-Numerierung) den 455 bis 477-Bereich der 16S-rRNA umfaßt.

2. Nucleinsäurefragment nach Anspruch 1, das unter den Bedingungen nicht hybridisieren kann mit rRNA oder die rRNA codierender DNA von Aeromonas sobria, Bacillus cereus, Bacillus subtilis, Brochothrix thermosphacta, Candida albicans, Citrobacter freundii, Corynebacterium xerosis, Corynebacterium diptheriae, Escherichia coli, Escherichia vulneris, Enterobacter agglomerans, Enterobacter cloacae, Klebsiella pneumoniae, Klebsiella oxytoca, Lactobacillus casei, Listeria monocytogenes, Pseudomonas aeruginosa, Rhodococcus egii, Salmonella arizonae, Salmonella cholerae-suis, Salmonella typhi, Serratia odorifera, Shigella boydii, Shigella flexneri, Shigella sonnei, Staphylococcus aureus, Staphylococcus epidermidis, Staphylococcus hominis, Staphylococcus saprophyticus, Streptococcus agalactiae, Streptococcus bovis, Streptococcus faecalis, Streptococcus faecium, Streptococcus lactis, Streptococcus mutans, Streptococcus pneumoniae oder Streptomyces globisporus.

3. Nucleinsäurefragment nach Anspruch 1 oder 2, das unter Hybridisierungsbedingungen hybridisieren kann mit Sonde 860, 1062 oder einem 880 umfassenden Oligomer mit einer Länge, größer als 880 aber kleiner als 1062, oder mit einer Sequenz, die komplementär zu einer der vorangehenden ist.

4. Nucleinsäurefragment nach Anspruch 1 oder 2, das unter Hybridisierungsbedingungen hybridisieren kann mit Sonde 926, 1111, 1112, 1113, 1063 oder einer Sequenz, die komplementär zu einer der vorangehenden ist.

5. Nucleinsäurefragment nach einem der vorangehenden Ansprüche, wobei die 16S-rRNA-Sequenz von Yersinia enterocolitica entweder:
(a) den 449 bis 484-Bereich von Y. enterocolitica 9610, nämlich: 5'-GGAAGGCCAAUAACUUAAUACGUUGUUGGAUUGACG-3'; oder
(b) den 449 bis 484-Bereich von Y. enterocolitica 954, nämlich: 5'-GGAAGGCAUAAAGGUUAAUAACCUUUGUGAUUGACG-3' umfaßt.

6. Satz von Nucleinsäurefragmenten, umfassend:
a) ein erstes Nucleinsäurefragment gemäß der Definition in Anspruch 1, das mit rRNA eines ersten Satzes von Yersinia enterocolitica hybridisieren kann und nicht mit rRNA eines zweiten Satzes von Yersinia enterocolitica oder mit rRNA von nicht-Yersinia enterocolitica; und
b) ein zweites Nucleinsäurefragment, ebenso gemäß der Definition in Anspruch 1, aber unterschiedlich von dem in (a), das mit rRNA des zweiten Satzes von Yersinia enterocolitica hybridisieren kann und nicht mit rRNA von nicht-Yersinia enterocolitica.

7. Satz von Nucleinsäurefragmenten nach Anspruch 6, wobei die Frragmente zusammengenommen mit mindestens 95 % von Yersinia enterocolitica-Arten hybridisieren können.

8. Satz von Nucleinsäurefragmenten nach Anspruch 6 oder 7, wobei:
(a) das erste Fragment mit dem 449 bis 484-16S-rRNA-Bereich von Y. enterocolitica 9610 (Sequenz A) hybridisieren kann, aber nicht mit dem 449 bis 484-16S-rRNA-Bereich von Y. enterocolitica 954 (Sequenz B); und
(b) das zweite Fragment mit Sequenz B, aber nicht mit Sequenz A hybridisieren kann.

9. Satz von Nucleinsäurefragmenten nach Anspruch 8, wobei das erste Fragment Sonde 1062 und das zweite Fragment 1063 umfaßt.

10. Verfahren zum Nachweis der Gegenwart von Yersinia enterocolitica in einer Probe, umfassend:
(a) Inkontaktbringen eines Nucleinsäurefragments gemäß der Definition in einem der Ansprüche 1 bis 5 oder eines Satzes von Nucleinsäurefragmenten nach einem der Ansprüche 6 bis 9 mit der Probe unter Bedingungen, die es mindestens einem der Fragmente erlauben, mit rRNA von Yersinia enterocolitica, falls in der Probe vorhanden, unter Bildung von Hybridnucleinsäurekomplexen zu hybridisieren, und
(b) Nachweis der Hybridkomplexe als Anzeichen für die Gegenwart von Yersinia enterocolitica in der Probe.

11. Verfahren nach Anspruch 10, wobei das Nucleinsäurefragment unter Hybridisierungsbedingungen hybridisieren kann mit Sonde 926, 1111, 1112, 1113, 1063 oder einer Sequenz, die komplementär zu einer der vorangehenden ist.

12. Verfahren nach Anspruch 9, wobei die Fragmente ein Gemisch umfassen von mindestens einem Fragment, das unter stringenten Bedingungen mit Sonde 926, 1111, 1112, 1113, 1063 oder einer Sequenz, die komplementär zu den vorangehenden ist, hybridisieren kann und mindestens einem weiteren Fragment, das unter stringenten Bedingungen mit Sonde 926, 1111, 1112, 1113, 1063 oder einer Sequenz, die komplementär zu diesen ist, hybridisieren kann.

13. Testkit zum Nachweis von Yersinia enterocolitica, umfassend ein Nucleinsäurefragment nach einem der Ansprüche 1 bis 5 oder einen Satz von Nucleinsäurefragmenten nach einem der Ansprüche 6 bis 9, gepackt in mindestens einem Behälter, und Instruktionen für die Verwendung des(der) Nucleinsäurefragment(e) zum Nachweis von Yersinia enterocolitica.

## Revendications

1. Fragment d'acide nucléique capable de s'hybrider, dans des conditions de stringence prédéterminées, à une séquence d'ARNr, ou à l'ADN codant pour ledit ARNr, présent(e) dans Yersinia enterocolitica et non à l'ARNr, ou à l'ADN codant pour ledit ARNr, de non-Yersinia enterocolitica, la séquence d'ARNr de Yersinia enterocolitica comprenant (en utilisant la convention de numérotation d'E. coli) la région 455 à 477 de l'ARNr 16S.

2. Fragment d'acide nucléique selon la revendication 1, qui n'est pas capable de s'hybrider, dans lesdites conditions, à l'ARNr, ou à l'ADN codant pour ledit ARNr, d'Aeromonas sobria, Bacillus cereus, Bacillus subtilis, Brochothrix thermosphacta, Candida albicans, Citrobacter freundii, Corynebacterium xerosis, Corynebacterium diptheriae, Escherichia coli, Escherichia vulneris, Enterobacter agglomerans, Enterobacter cloacae, Klebsiella pneumoniae, Klebsiella oxytoca, Lactobacillus casei, Listeria monocytogenes, Pseudomonas aeruginosa, Rhodococcus egii, Salmonella arizonae, Salmonella cholerae-suis, Salmonella typhi, Serratia odorifera, Shigella boydii, Shigella flexneri, Shigella sonnei, Staphylococcus aureus, Staphylococcus epidermidis, Staphylococcus hominis, Staphylococcus saprophyticus, Streptococcus agalactiae, Streptococcus bovis, Streptococcus faecalis, Streptococcus faecium, Streptococcus lactis, Streptococcus mutans, Streptococcus pneumoniae, ou Streptomyces globisporus.

3. Fragment d'acide nucléique selon la revendication 1 ou 2, qui est capable de s'hybrider, dans des conditions d'hybridation, à la sonde 880, 1062, ou à un oligomère comprenant 880 et ayant une longueur supérieure à 880, mais inférieure à 1062, ou à une séquence complémentaire de l'une quelconque des séquences précédentes.

4. Fragment d'acide nucléique selon la revendication 1 ou 2, qui est capable de s'hybrider, dans des conditions d'hybridation, à la sonde 926, 1111, 1112, 1113, 1063, ou à une séquence complémentaire de l'une quelconque des séquences précédentes.

5. Fragment d'acide nucléique selon l'une quelconque des revendications précédentes, dans lequel la séquence d'ARNr 16S de Yersinia enterocolitica comprend l'une ou l'autre parmi :
(a) la région 449 à 484 de Y. enterocolitica 9610, qui est : 5'-GGAAGGCCAAUAACUUAAUACGUUGUUGGAUUGACG-3' ; et
(b) la région 449 à 484 de Y. enterocolitica 954, qui est :

5'-GGAAGGCAUAAAGGUUAAUAACCUUUGUGAUUGACG-3'.

6. Jeu de fragments d'acide nucléique comprenant :
(a) un premier fragment d'acide nucléique, tel que défini à la revendication 1, qui est capable de s'hybrider à l'ARNr d'un premier jeu de Yersinia enterocolitica et non à l'ARNr d'un second jeu de Yersinia enterocolitica ou à l'ARNr de non-Yersinia enterocolitica ; et
(b) un second fragment d'acide nucléique, également tel que défini à la revendication 1, mais différent de celui dans (a), qui est capable de s'hybrider à l'ARNr dudit second jeu de Yersinia enterocolitica et non à l'ARNr de non-Yersinia enterocolitica.

7. Jeu de fragments d'acide nucléique selon la revendication 6, dans lequel les fragments sont ensemble capables de s'hybrider à au moins 95% des espèces Yersinia enterocolitica.

8. Jeu de fragments d'acide nucléique selon la revendication 6 ou 7, dans lequel :
(a) le premier fragment est capable de s'hybrider à la région 449 à 484 de 1'ARNr 16S de Y. enterocolitica 9610 (séquence A), mais non à la région 449 à 484 de l'ARNr 16S de Y. enterocolitica 954 (séquence B) ; et
(b) le second fragment est capable de s'hybrider à la séquence B, mais non à la séquence A.

9. Jeu de fragments d'acide nucléique selon la revendication 8, dans lequel le premier fragment comprend la sonde 1062 et le second fragment comprend la sonde 1063.

10. Procédé pour détecter la présence de Yersinia enterocolitica dans un échantillon, le procédé comprenant les opérations consistant à :
(a) mettre en contact un fragment d'acide nucléique tel que défini à l'une quelconque des revendications 1 à 5 ou un jeu de fragments d'acide nucléique tel que défini à l'une quelconque des revendications 6 à 9, avec ledit échantillon, dans des conditions qui permettent au moins à l'un desdits fragments de s'hybrider à l'ARNr de Yersinia enterocolitica, s'il est présent dans ledit échantillon, pour former des complexes d'acide nucléique hybrides ; et
(b) détecter les complexes hybrides en tant qu'indication de la présence de Yersinia enterocolitica dans l'échantillon.

11. procédé selon la revendication 10, dans lequel le fragment d'acide nucléique est capable de s'hybrider, dans des conditions d'hybridation, à la sonde 926, 1111, 1112, 1113, 1063, ou à une séquence complémentaire de l'une des séquences précédentes.

12. Procédé selon la revendication 9, dans lequel les fragments comprennent un mélange d'au moins un fragment capable de s'hybrider, dans des conditions stringentes, à la sonde 926, 1111, 1112, 1113, 1063, ou à une séquence complémentaire de l'une quelconque des séquences précédentes ; et d'au moins un autre fragment capable de s'hybrider, dans des conditions stringentes, à la sonde 926, 1111, 1112, 1113, 1063, ou à une séquence complémentaire de l'une quelconque de ces séquences,

13. Coffret d'essai pour détecter Yersinia enterocolitica, comprenant un fragment d'acide nucléique tel que défini à l'une des revendications 1 à 5 ou un jeu de fragments d'acide nucléique tels que définis à l'une des revendications 6 à 9, conditionné dans au moins un récipient, et des instructions pour utiliser ledit (ou lesdits) fragment(s) d'acide nucléique pour la détection de Yersinia enterocolitica.
